(19) **Europäisches Patentamt**

**European Patent Office**

**Office européen des brevets**

(11) **EP 1 291 037 B1**

(12) **EUROPÄISCHE PATENTSCHRIFT**

(45) Veröffentlichungstag und Bekanntmachung des Hinweises auf die Patenterteilung:
**21.06.2006 Patentblatt 2006/25**

(21) Anmeldenummer: **02090320.9**

(22) Anmeldetag: **05.09.2002**

(51) Int Cl.:
*A61N 1/368* (2006.01)　　*A61N 1/362* (2006.01)
*A61N 1/365* (2006.01)　　*A61B 5/0432* (2006.01)
*A61B 5/0468* (2006.01)　　*A61N 1/37* (2006.01)

(54) **Ratenadaptiver Herzschrittmacher**

Rate adaptive pacemaker

Stimulateur cardiaque à fréquence asservie

(84) Benannte Vertragsstaaten:
**AT BE BG CH CY CZ DE DK EE ES FI FR GB GR IE IT LI LU MC NL PT SE SK TR**

(30) Priorität: **06.09.2001 DE 10144442**

(43) Veröffentlichungstag der Anmeldung:
**12.03.2003 Patentblatt 2003/11**

(73) Patentinhaber: **Biotronik GmbH & Co. KG**
**12359 Berlin (DE)**

(72) Erfinder:
- **Mlynski, Michael Frank**
  **52078 Aachen (DE)**
- **Diem, Björn Henrik**
  **52064 Aachen (DE)**

- **Ameling, Walter**
  **52074 Aachen (DE)**
- **Hanrath, Peter**
  **52074 Aachen (DE)**

(74) Vertreter: **Lindner-Vogt, Karin L.**
**BIOTRONIK GmbH & Co. KG**
**Corporate Intellectual Properties**
**Woermannkehre 1**
**12359 Berlin (DE)**

(56) Entgegenhaltungen:
**EP-A- 0 448 193　　　WO-A-01/05465**
**US-A- 5 144 949　　　US-A- 5 292 340**
**US-A- 5 514 164　　　US-A- 5 792 192**

**Beschreibung**

Um Herzschrittmacher patienten-individuell programmieren und so für jeden Patienten auf die kardiale Erkrankung des Patienten abgestimmte Therapien schaffen zu können, verfügen Herzschrittmacher (HSM) heutzutage über eine Vielzahl von Parametern, mittels der die Arbeitsweise des Herzschrittmachers und damit die vom Herzschrittmacher im Betrieb gebotene Therapie eingestellt werden kann.

Bei der Implantation werden diese Parameter erstmals patienten-individuell programmiert. Danach kommt der Patient in regelmäßigen Abständen (typisch alle 6 Monate) zu den sogenannten Nachsorgeuntersuchung, bei der nicht nur die Batterie und die eingestellten Pacing- und Sensingschwellen überprüft werden, sondern u.U. auch Parameter hinsichtlich eines sich geänderten Krankheitsbildes neu eingestellt werden.

Durch dieses Vorgehen ist es zwar möglich, patientenindividuelle Therapien zu programmieren, jedoch sind diese für den folgenden Zeitraum bis zur nächsten Nachsorgeuntersuchung fest eingestellt. Aus diesem Grund wird von den Herzschrittmacher-Herstellern bereits seit längerer Zeit versucht, die Parameter kontinuierlich an die aktuellen Bedürfnisse des Patienten automatisch anzupassen.

Es sind viele ratenadaptive Herzschrittmacher bekannt, bei denen eine große Vielzahl von physiologischen Parametern als Indikator des metabolischen Bedarfs erfasst und zur Steuerung der Ratenadaption verwendet werden.

Aus der DE 199 40 952 ist ein ratenadaptiver Herzschrittmacher bekannt, bei dem die Ratenadaption in Abhängigkeit von der Respirationsrate erfolgt. Den Ansatzpunkt stellt hierbei die zentralnervöse Kopplung von Respirations- und Kreislauftätigkeit dar, bei der als kreislaufrelevante Größen vor allem die Herzfrequenz, das Herzschlagvolumen und die Vasomotorik von Bedeutung sind. Die Messung der Respirationsrate kann durch Messungen der intrathorakalen und/oder der intrakardialen Impedanz erfolgen.

Die EP 0 449 401 zeigt ebenfalls einen ratenadaptiven Herzschrittmacher, welcher ein Impedanzsystem zur Messung eines rechtsventrikulären (oder atrialen) Volumens oder einen Druckwandler zur Messung des rechtsventrikulären (oder atrialen) Druckes und eine Signalverarbeitungseinheit zum schlagweisen ("beat-to-beat") Extrahieren eines der Volumen- oder Druckparameter aufweist, um dadurch ein Signal zu erhalten, welches mit der Respirationsrate variiert, und eine Spitze-Spitze-Amplitude proportional zu der Respirationstiefe aufweist. Die Signalverarbeitungseinheit ist dabei dazu vorgesehen, die Periode des Respirationssignals zu extrahieren und die Spitze-Spitze-Amplitude sowie die resultierenden Signalen dazu zu verwenden, die erforderliche Stimulationsrate für den Herzschrittmacher zu bestimmen.

Algorithmen zur Stabilisierung der Herzfrequenz sind beispielsweise aus PA-CE, Vol. 23, Oktober 2000, Part I, Seite 1509 ff. bekannt.

Es ist wünschenswert, die Pacing- und Sensingeigenschaften von Herzschrittmachern noch weiter an den tatsächlichen metabolischen Bedarf eines Patienten anpassen zu können und/oder die Herzfrequenz zu stabilisieren.

Es ist daher Aufgabe der Erfindung die Ratenadaptionseigenschaften von ratenadaptiven Herzschrittmachern zu verbessern.

Die Aufgabe der Erfindung wird durch einen ratenadaptiven Herzschrittmacher mit den Merkmalen des Anspruchs 1 gelöst.

Erfindungsgemäß ist dazu ein ratenadaptiver Herzschrittmacher vorgesehen, welcher eine Erfassungseinheit, eine Ereignis-Bestimmungseinheit, eine Stimulations-Steuereinheit und eine Stimulationseinheit aufweist, wobei die Ereignis-Bestimmungseinheit mit der Erfassungseinheit und Stimulations-Steuereinheit verbunden sowie die Stimulationseinheit mit der Stimulations-Steuereinheit verbunden ist. Die Erfassungseinheit erfasst EKG-Signale eines Herzens und die Ereignis-Bestimmungseinheit bestimmt anhand der von der Erfassungseinheit erfassten EKG-Signale und von der Stimulations-Steuereinheit ausgesendeten Signalen, ob ein erfasstes Ereignis ein spontanes oder ein stimuliertes Ereignis darstellt. Die Stimulations-Steuereinheit ermittelt eine Stimulationsrate in Abhängigkeit von einer Grundrate und einem physiologischen Bedarf und verändert die Grundrate in Abhängigkeit von der Ereignis-Bestimmungseinheit in einem ersten Modus bei Auftreten von atrialen oder ventrikulären Ereignissen derart, dass im Falle eines spontanen atrialen Ereignisses ein Spontanintervall um einen ersten Änderungswert verlängert wird, dass im Falle eines stimulierten atrialen Ereignisses ein Grundintervall um einen zweiten Änderungswert verlängert wird und dass im Falle einer ventrikulären Extrasystole ein Grundintervall um einen dritten Änderungswert verlängert wird, wodurch in allen Fällen die Grundrate gesenkt wird. Alternativ oder zusätzlich ist die Stimulations-Steuereinheit ausgebildet, die Grundrate in einem zweiten Modus bei Auftreten von ventrikulären Ereignissen derart zu verändern, dass im Falle eines spontanen ventrikulären Ereignisses die Grundrate erhöht wird und im Falle eines stimulierten ventrikulären Ereignisses die Grundrate gesenkt wird. Das Absenken der Grundrate im zweiten Modus erfolgt schlagweise in kleineren Schritten, als das ebenfalls schlagweise Erhöhen der Grundrate im Falle erfasster natürlicher (spontaner) ventrikulärer Ereignisse. Die Stimulationseinheit stimuliert das Herz entsprechend den von der Stimulations-Steuereinheit empfangenen Signalen.

Die mit der Erfindung einhergehenden Vorteile liegen insbesondere darin, dass eine Umsetzung von medizinischem Expertenwissen in eine für einen Herzschrittmacher verarbeitbare Form, d.h. minimaler Rechenaufwand für den Herzschrittmacher und damit Erfüllung einer Umsetzbarkeit, ohne die (Batterie-) Lebenszeit wesentlich zu beeinträchtigen, erreicht wird.

Einhergehend mit einer permanenten und sofortigen Therapieoptimierung (Beat-To-Beat-Algorithmen) erhält der Herzschrittmacher-Patient eine u.U. deutliche Erhöhung der Lebensqualität. Die Therapieverbesserungen für den Patienten sind durch extrakorporale Überwachungen (z.B. Langzeit-Oberflächen-EKG) nachvollziehbar (jede Herzaktivität und damit auch die Algorithmus-Funktionalität kann genau analysiert werden).

Bei einer bevorzugten Ausgestaltung der Erfindung ist die Stimulations-Steuereinheit dazu geeignet, die Grundrate des Herzens innerhalb vorgegebener Grenzen zu bestimmen. Damit wird gewährleistet, dass die Grundrate des Herzens nicht unter einen kritischen Wert sinkt bzw. über einen kritischen Wert steigt, welcher das Wohlbefinden oder die Gesundheit des Patienten beeinträchtigen kann.

Bei einer weiteren Ausgestaltung der Erfindung weist der ratenadaptive Herzschrittmacher eine Aktivitäts-Erfassungseinheit auf, welche dazu ausgebildet ist, die Aktivität eines Patienten zu erfassen und ein entsprechendes Aktivitätssignal auszugeben. Die Stimulations-Steuereinheit ist mit der Aktivitäts-Erfassungseinheit verbunden und dazu ausgebildet, eine Grundrate eines Patienten entsprechend einer ersten oder einer zweiten Betriebsart derart ratenadaptiv zu bestimmen, dass die Grundrate des Herzens entsprechend der ersten Betriebsart in einem vorgegebenen ersten Schrittintervall abgesenkt wird, wenn innerhalb eines vorgegebenen ersten Zeitintervalls das Aktivitätssignal keine Aktivität des Patienten anzeigt, und die Grundrate des Herzens entsprechend der zweiten Betriebsart in einem vorgegebenen zweiten Schrittintervall erhöht wird, wenn innerhalb eines vorgegebenen Schrittintervall erhöht wird, wenn innerhalb eines vorgegebenen zweiten Zeitintervalls das Aktivitätssignal eine Aktivität des Patienten anzeigt. Das erste Zeitintervall ist dabei länger als das zweite Zeitintervall, während das erste Schrittintervall identisch zum zweiten Schrittintervall ist.

Neben der ratenadaptiven Steuerung des Herzschrittmachers entsprechend atrialen oder ventrikulären Ereignissen berücksichtigt der Herzschrittmacher somit auch die Aktivität des Patienten. Bei Ruhe des Patienten wird die Grundfrequenz abgesenkt, während sie bei einer erneuten Aktivität wieder erhöht wird.

Die damit einhergehenden Vorteile liegen insbesondere darin, dass die Grundfrequenz nicht abrupt sondern schrittweise abgesenkt und bei Erfassung einer Aktivität des Patienten schnell wieder angehoben wird, so dass der metabolische Bedarf des Patienten besser und situations- bzw. belastungsbedingt gedeckt werden kann.

Weitere Ausführungen der Erfindung sind Gegenstand der Unteransprüche.

Nachfolgend werden Ausführungsbeispiele der Erfindung unter Bezugnahme auf die Zeichnung näher erläutert. Dabei zeigen:

Fig. 1    ein Blockschaltbild eines ratenadaptiven Herzschrittmacher gemäß einem ersten und einem zweiten Ausführungsbeispiel,

Fig. 2    einen Graph der Herzfrequenz eines Patienten, mit einem ratenadaptiven Herzschrittmacher gemäß einem ersten Ausführungsbeispiel von Figur 1, über die Zeit,

Fig. 3    einen Graph der Herzfrequenz eines Patienten, mit einem ratenadaptiven Herzschrittmacher gemäß einem zweiten Ausführungsbeispiel von Figur 1, über die Zeit,

Fig. 4    ein Blockschaltbild eines ratenadaptiven Herzschrittmacher gemäß einem dritten Ausführungsbeispiel,

Fig. 5    ein Flussdiagramm zur Steuerung des ratenadaptiven Herzschrittmachers gemäß dem dritten Ausführungsbeispiel in Fig. 4,

Fig. 6    einen Graph der Herzfrequenz eines Patienten, mit einem ratenadaptiven Herzschrittmacher gemäß dem dritten Ausführungsbeispiel von Figur 4, über die Zeit,

Fig. 7    ein Blockschaltbild eines ratenadaptiven Herzschrittmacher gemäß einem vierten Ausführungsbeispiel,

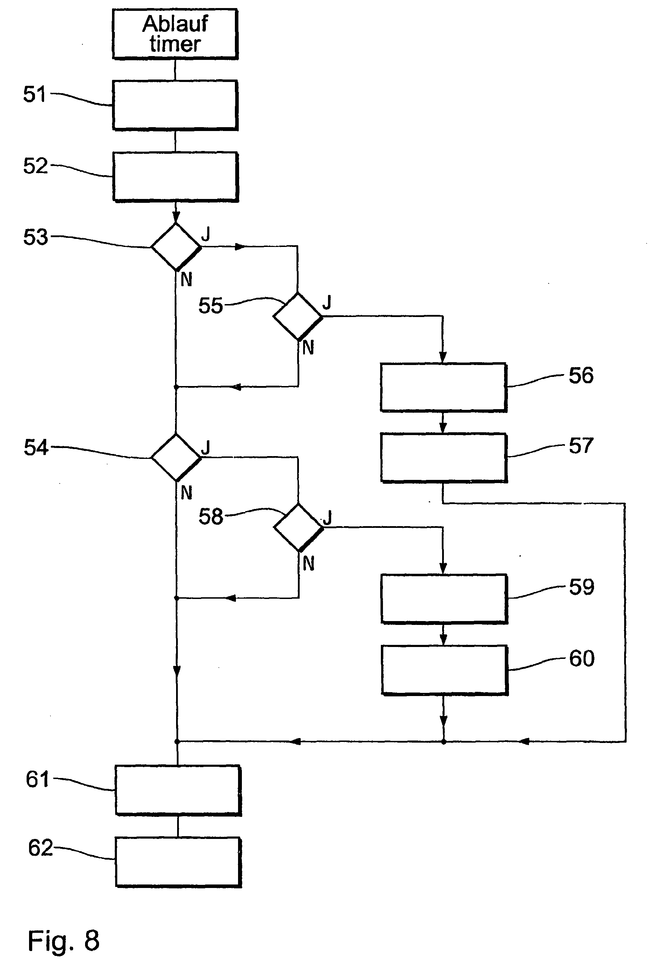

Fig. 8    ein Flussdiagramm zur Steuerung des ratenadaptiven Herzschrittmachers gemäß dem vierten Ausführungsbeispiel in Fig. 6, und

Fig. 9    einen Graph der Herzfrequenz eines Patienten, mit einem ratenadaptiven Herzschrittmacher gemäß einem vierten Ausführungsbeispiel von Figur 7, über die Zeit.

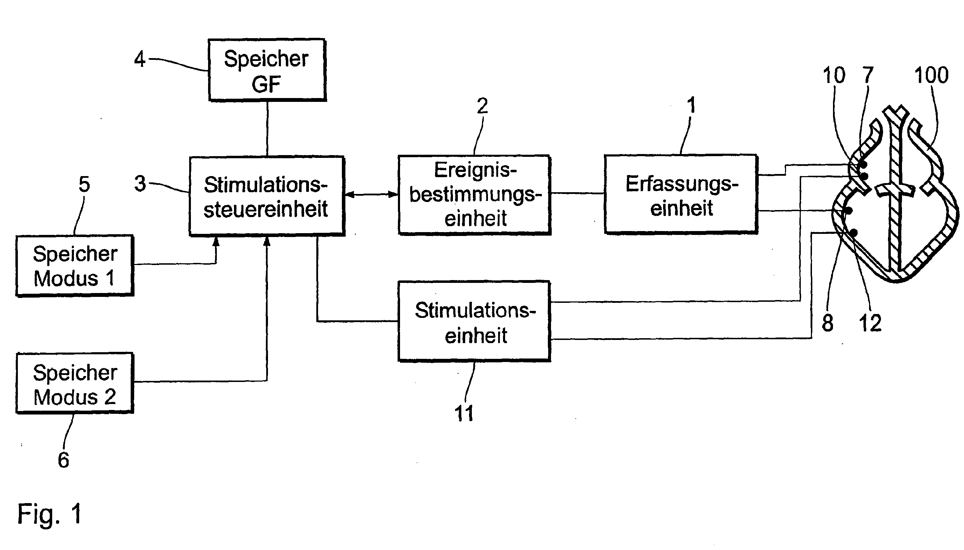

Fig. 1 zeigt einen ratenadaptiven Herzschrittmacher gemäß einem ersten und zweiten Ausführungsbeispiel. Der Herzschrittmacher weist dabei eine Erfassungseinheit 1, eine Ereignis-Bestimmungseinheit 2, eine Stimulations-Steuereinheit 3, einen Grundfrequenzparameter-Speicher 4, einen Speicher 5 für den Modus 1, einen Speicher 6 für den Modus 2 und eine Stimulationseinheit 11 auf.

Die Erfassungseinheit 1 ist einerseits mit zwei Elektroden verbunden, von denen eine Elektrode 7 im Atrium und eine Elektrode 8 im Ventrikel, und andererseits mit der Ereignis-Bestimmungseinheit 2. Die Stimulations-Steuereinheit 3 ist mit der Ereignis-Bestimmungseinheit 2, dem Grundfrequenzparameter-Speicher 4, dem Speicher 5, dem Speicher 6 sowie mit der Stimulationseinheit 11 verbunden. Die Stimulationseinheit 11 ist mit Elektroden 10 und 12 im Atrium bzw. Ventrikel verbunden. Es sind verschiedene Zuordnungen jeweils einer oder mehrer Elektroden in Atrium und Ventrikel zu der Erfassungseinheit 1 und der Stimulationseinheit 11 ggf. unter Einsatz von Umschaltern möglich, so dass an sich bekanntes uni- oder bipolares Sensing und/oder eine uni- oder bipolare Stimulation möglich sind.

Die Erfassungseinheit 1 erfasst die EKG-Signale des Herzens 100 anhand der Elektroden 7 und 8 und leitet diese Signale an die Ereignis-Bestimmungseinheit 2 weiter. Die Ereignis-Bestimmungseinheit 2 empfängt die von der Erfassungseinheit 1 ausgegebenen EKG-Signale des Herzens sowie von der Stimulations-Steuereinheit 3 ausgegebene Signale, welche anzeigen, ob eine Stimulation stattgefunden hat. Anhand dieser empfangenen Signale bestimmt die Ereignis-Bestimmungseinheit 2, ob es sich bei einem erfassten Ereignis um ein spontanes Ereignis oder um ein stimuliertes Ereignis handelt. Das Ergebnis dieser Bestimmung wird an die Stimulations-Steuereinheit 3 weitergeleitet. Anhand dieses Ergebnisses wählt die Stimulations-Steuereinheit 3 den im Speicher 5 gespeicherten Modus 1 oder den im Speicher 6 gespeicherten Modus 2 aus.

In dem Speicher 4 wird die patientenspezifische obere und untere Grenze der Grundfrequenz $GF_{OG}$ und $GF_{UG}$ sowie bei Bedarf die variable untere Grundfrequenz $GF_{UGvar}$ für den Herzschrittmacher gespeichert.

Mit Hilfe des ersten Ausführungsbeispiels des in Fig. 1 gezeigten ratenadaptiven Herzschrittmachers soll die Grundfrequenz GF möglichst dicht unter der Eigenfrequenz des Patienten gehalten werden. Das ersten Ausführungsbeispiel entspricht dabei dem Modus 1. Dadurch greift der Herzschrittmacher HSM standardmäßig nicht ein, es werden jedoch kompensatorische Pausen nach ventrikulären Extrasystolen VES durch einen HSM-Stimulus unterbrochen. Im wesentlichen soll dabei Ap/(Ap+As) ≈ VES-Anteil entsprechen.

Geeignete Patienten sind bevorzugt Patienten mit einem Herzschrittmacher im DDD oder AAI-Modus und mit häufigen ventrikulären und supraventrikulären Extrasystolen (z.B. bei Sick-Sinus-Syndrom, AV-Blockierungen oder HOCM). Dies lässt sich beispielsweise bei Patienten beispielsweise mit einem AV-Block II°, Typ 2:1, dominierend, mit häufigen VES einsetzen.

Nicht geeignet ist ein Herzschrittmacher gemäß dem ersten Ausführungsbeispiel jedoch für Patienten mit höher eingestellter Grundfrequenz, z.B. nach AV-Knotenablation, Patienten mit hochgradiger Herzinsuffizienz ohne Schenkelblock, Patienten, bei denen ein hoher Stimulationsanteil gewünscht wird (z.B. bei Torsaden) und Patienten mit einem Herzschrittmacher mit einem VDD- und VVI-Modus, da es hier bei erhaltenem Vorhofrhythmus zur asynchronen Ventrikelstimulation kommt.

Ein Nachführen der Grundfrequenz an die aktuelle intrinsische Herzfrequenz soll die kompensatorischen Pausen verringern ohne in größeren Maße den intrinsischen Herzfrequenzverlauf zu stören. Dies ist besonders bei den Patienten interessant, bei denen eine niedrige Grundfrequenz programmiert wurde und hierdurch lange kompensatorische Pausen entstehen können, ohne dass der Schrittmacher einschreiten kann.

Die Grundfrequenz wird so nachgeführt, dass sie möglichst knapp unterhalb der intrinsischen Herzfrequenz liegt. Insgesamt sollte - ausgenommen in den kompensatorischen Pausen nach Extrasystolen - möglichst wenig stimuliert werden. Gleichzeitig wird dafür gesorgt, dass die Grundfrequenz GF stets innerhalb der definierten Grenzen Grundfrequenz UG, d. h. der unteren Grundfrequenz, und Grundfrequenz OG, d. h. der oberen Grundfrequenz, liegt.

Folgende Eingangswerte müssen von einem Arzt vorgeben werden:

| | |
|---|---|
| $GF_{UG}$ | *Minimal erlaubter Wert für die Grundfrequenz (untere Grenze)* |
| $GF_{OG}$ | *Maximal erlaubter Wert für die Grundfrequenz (obere Grenze)* |
| *delta_ap* | *Änderungswert nach Ap-Event (einem atrialen Pacing- bzw. Stimulations-Ereignis)* |
| *delta_as* | *Änderungswert nach As-Event (einem atrialen spontanen Ereignis)* |
| *delta_ves* | *Änderungswert nach VES-Event* |

Hilfswerte (interne Variablen):

| | |
|---|---|
| $GI_{UG}$ | *Minimal erlaubter Wert für das Grundintervall (untere Grenze)* |
| $GI_{OG}$ | *Maximal erlaubter Wert für das Grundintervall (obere Grenze)* |
| *GI* | *Grundintervall* |
| *ASI* | *Letztes atriales Spontanintervall (intrinsisch und/oder stimuliert)* |
| *V_VES_I* | *Intervall zwischen letztem ventr. Ereignis (intr. oder stimuliert) und detektierter VES* |
| *AV-Delay* | *Herzschrittmacher-Parameter* |

Zur Initialisierung wird

$$GI_{OG} = 60000 / GF_{UG}$$

$$GI_{UG} = 60000 / GF_{OG}$$

gesetzt.

Nach jeder Vorhofaktion wird die folgende Auswertung gestartet:

$$Falls\ As \quad \Rightarrow \quad GI = ASI + delta\_as \qquad (spontanes\ atriales\ Ereignis)$$

$$Falls\ Ap \quad \Rightarrow \quad GI = GI + delta\_ap \qquad (stimuliertes\ atriales\ Ereignis)$$

$$Falls\ VES \quad \Rightarrow \quad GI = AV\text{-}Delay + V\_VES\_I + delta\_ves$$

$$Falls\ (GI < GI_{UG}) \qquad GI = GI_{UG}$$

$$Falls\ (GI > GI_{OG}) \qquad GI = GI_{OG}$$

Wenn ein atriales Stimulations-Ereignis von der Erfassungseinheit 1 erfasst und von der Ereignis-Bestimmungseinheit 2 bestimmt wird, wird das Grundintervall GI um den Änderungswert delta_ap nach dem $A_P$-Ereignis verlängert. Wenn ein atriales spontanes Ereignis von der Erfassungseinheit 1 hingegen erfasst und von der Ereignis-Bestimmungseinheit 2 bestimmt wird, wird das Spontanintervall um den Änderungswert delta_as nach dem $A_S$-Ereignis verlängert.

Anschließend wird überprüft, ob das Grundintervall GI sich oberhalb des maximal erlaubten Wertes für das Grundintervall $GI_{OG}$ oder unterhalb des minimal erlaubten Wertes für das Grundintervall $GI_{UG}$ befindet. Wenn dies der Fall ist, wird das Grundintervall GI auf den minimal erlaubten bzw. den maximal erlaubten Wert für das Grundintervall hoch- bzw. runtergesetzt. Dieser Algorithmus wird nach jeder Vorhofaktion, d. h. nach einem Stimulations-Ereignis oder nach einem spontanen Ereignis, durchgeführt.

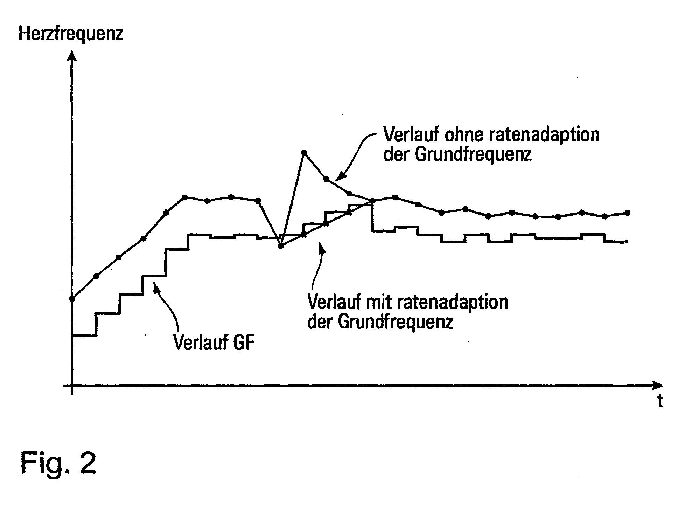

Fig. 2 zeigt einen Graph der Herzfrequenz über die Zeit. In Fig. 2 sind sowohl der Verlauf der Grundfrequenz GF als auch der Verlauf der Herzfrequenz dargestellt, und zwar sowohl für den Fall ohne Ratenadaption der Grundfrequenz als auch für den Fall der ratenadaptiven Nachführung der Grundfrequenz. Das zweite Ausführungsbeispiel entspricht dabei dem Modus 2. Der Verlauf des Graph zeigt anschaulich, dass bei einer VES die kompensatorische Pause auf Grund der nachgeführten Grundfrequenz GF durch einen Stimulus unterbrochen wird.

Mit Hilfe des zweiten Ausführungsbeispiels des in Fig. 1 gezeigten ratenadaptiven Herzschrittmachers soll durch eine permanente Stimulation im Ventrikel die Überleitung schneller atrialer Schläge durch retrograde Blockierungen des AV-Knoten unterdrückt werden. Gleichzeitig soll die Grundfrequenz GF jedoch nicht auf einem unnötigen hohen Wert liegen, sondern situationsangepasst herabgesenkt werden können.

Dies lässt sich bevorzugt bei Patienten beispielsweise mit chronisches Vorhofflimmern und VVI-Modus einsetzen.

Nicht geeignet ist ein Herzschrittmacher gemäß dem zweiten Ausführungsbeispiel hingegen für Patienten mit erhaltenem Vorhofrhythmus oder nur intermitt. Vorhofflimmern, bei Patienten mit hoch eingestellter Grundfrequenz, z.B. nach AV-Knotenablation, bei einem HSM im VDD- und DDD-Modus, da es hier bei erhaltenem Vorhofrhythmus zur vermehrten asynchronen Ventrikelstimulation kommt und einem HSM im AAI-Modus, da keine Daten über Ventrikelereignisse vorliegen.

Es wurde gezeigt, dass bei Patienten mit chronischem Vorhofflimmern eine Stimulationsart, bei der über 90% der ventrikulären Aktionen stimuliert sind, zu einer Stabilisierung der Herzfrequenz mit Unterdrückung schneller übergeleiteter Schläge führt.

Die Grundfrequenz GF wird derart nachgeführt, dass sie möglichst knapp oberhalb der intrinsischen Herzfrequenz liegt. Im Wesentlichen soll dabei Vp/(Vp+Vs) ≈ 100% entsprechen. Gleichzeitig steuert die Stimulations-Steuereinheit 3 die Stimulationseinheit 11 entsprechend den im Speicher 4 gespeicherten Daten derart, dass die Grundfrequenz stets innerhalb der definierten Grenzen liegt, d. h. Grundfrequenz $GF_{UG}$ als minimal erlaubter Wert für die Grundfrequenz und Grundfrequenz $GF_{OG}$ als maximal erlaubter Wert für die Grundfrequenz.

Folgende Eingangswerte müssen von einem Arzt patientenspezifisch vorgeben werden:

$GF_{UG}$      *Minimal erlaubter Wert für die Grundfrequenz (untere Grenze)*
$GF_{OG}$      *Maximal erlaubter Wert für die Grundfrequenz (obere Grenze)*
*delta_vp*      *Änderungswert nach Vp-Event (z.B.1 ppm)*
*delta_vs*      *Änderungswert nach Vs-Event*
*n_limit*      *Obere Grenze für intemen Zähler (z.B. 10)*
*n_reinit*      *Re-Inltialisierung des internen Zählers (z.B. 7)*

Parameter:

*GF*      *Grundfrequenz*

Hilfswerte (interne Variablen):

*n*      *Zähler*

Zur Initialisierungen wird:

     *n = 0 gesetzt.*

Nach jeder Ventrikelaktion wird die folgende Auswertung gestartet:

*Falls Vp* $\Rightarrow$ *n++*      *(stimuliertes ventrikuläres Ereignis)*
     *if (n=n_limit) { GF = GF – delta_vp; n=n_reinit; }*
     *if ($GF< GF_{UG}$) { $GF = GF_{UG};$ }*

*Falls Vs* $\Rightarrow$ *n = 0*      *(spontanes ventrikuläres Ereignis)*
     *GF = GF + delta_vs*
     *if ($GF> GF_{OG}$) { $GF = GF_{OG};$ }*

Der Algorithmus gemäß dem zweiten Ausführungsbeispiel der Erfindung wird für jede Ventrikelaktion durchgeführt und entspricht dem Modus zwei:

Wenn ein ventrikuläres Stimulations-Ereignis $V_P$ von der Erfassungseinheit 1 erfasst und von der Ereignis-Bestimmungseinheit 2 bestimmt wird, wird der Zähler n - der bei der Initialisierung auf 0 gesetzt wurde - um 1 erhöht. Wenn der Zähler n den Wert n_limit, z.B. 10, erreicht hat, wird die Grundfrequenz GF um delta_vp, z.B. 1 ppm (Puls pro Minute), abgesenkt und der Zähler n wird auf n_reinit, z.B. 7, zurückgesetzt. Anschließend wird überprüft, ob die Grundfrequenz GF unterhalb dem minimal erlaubten Wert für die Grundfrequenz $GF_{UG}$ liegt. Wenn dies der Fall ist, wird die Grundfrequenz GF auf den minimal erlaubten Wert für die Grundfrequenz $GF_{UG}$ gesetzt.

Wenn ein spontanes ventrikuläres Ereignis $V_S$ von der Erfassungseinheit 1 erfasst und von der Ereignis-Bestimmungseinheit 2 bestimmt wird, wird der Zähler n auf 0 gesetzt und die Grundfrequenz GF wird um delta_vs, z.B. 4 ppm, erhöht. Anschließend wird überprüft, ob die Grundfrequenz GF oberhalb dem maximal erlaubten Wert für die Grundfrequenz $GF_{OG}$ liegt. Wenn dies der Fall ist, wird die Grundfrequenz GF auf den maximal erlaubten Wert für die Grundfrequenz $GF_{OG}$ gesetzt.

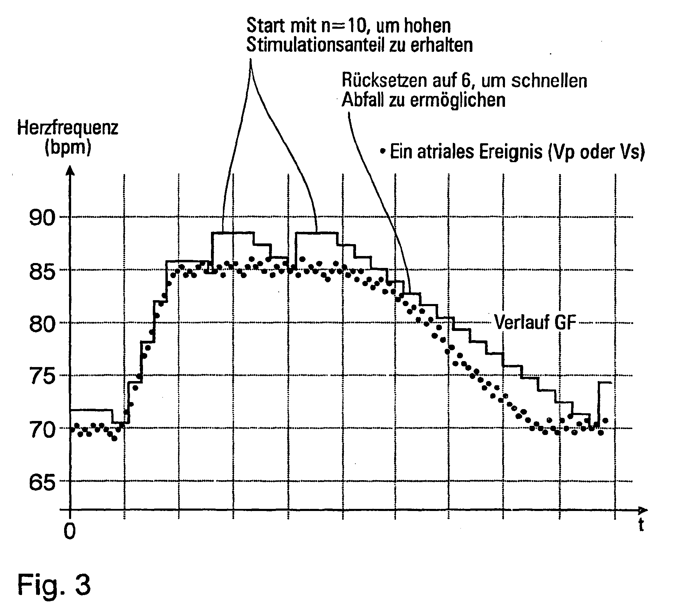

Fig. 3 zeigt einen Graph der Herzfrequenz über die Zeit. In Fig. 3 sind sowohl der Verlauf der Grundfrequenz GF als auch die ventrikulären Ereignisse dargestellt, wobei die ventrikulären Ereignisse als Punkte und der Verlauf der Grundfrequenz GF als durchgezogene Linie dargestellt werden. Der Verlauf des Graph zeigt anschaulich, wie die Grundfrequenz GF dem Verlauf der ventrikulären Ereignisse angepasst wird. Um einen hohen Stimulationsanteil zu erhalten, wird eine Absenkung des Stimulationsanteils erst initialisiert, wenn der Zähler n den Wert 10 erreicht hat. Das anschließende Rücksetzen des Zählers n auf 6 ermöglicht einen schnellen Abfall des Stimulationsanteils.

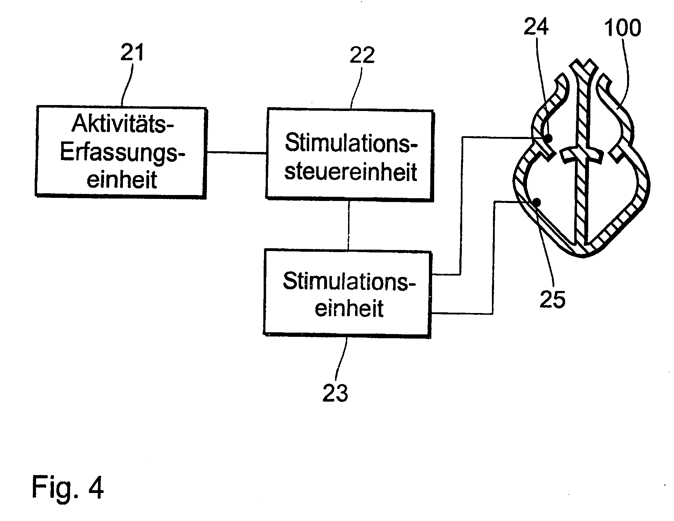

In Fig. 4 wird ein Herzschrittmacher gemäß einem dritten Ausführungsbeispiel der Erfindung gezeigt. Der Herzschrittmacher weist dabei eine Stimulations-Steuereinheit 22, eine Stimulationseinheit 23 sowie eine Aktivitäts-Erfassungseinheit 21 auf, wobei die Stimulations-Steuereinheit 22 mit der Stimulationseinheit 23 und der Aktivitäts-Erfassungseinheit 21 verbunden ist. Die Stimulationseinheit 23 ist wiederum mit der atrialen Elektrode 24 und/oder der ventrikulären Elektrode 25 verbunden und stimuliert das Herz entsprechend den von der Stimulations-Steuereinheit 22 erhaltenen Steuersignalen über diese Elektroden.

Die Aktivitäts-Erfassungseinheit 21 kann beispielsweise eine Beschleunigung-Messeinheit oder alternativ dazu ebenfalls eine Respirationsrate-Erfassungseinheit, wie in DE 197 47 820 oder EP 0 449 401 beschrieben, sein.

Die Nachteile einer zeitgesteuerten (Nacht-) Absenkung der Grundfrequenz GF bei Patientenruhe soll durch eine aktivitätsgesteuerte Absenkung ersetzt werden. Ist der Patient über einen längeren Zeitraum ruhig, so wird die Grundfrequenz schrittweise um insgesamt 10 ppm abgesenkt. Sobald eine Aktivität erkannt wird, wird die Grundfrequenz rasch wieder angehoben.

Der ratenadaptiven Herzschrittmacher gemäß dem dritten Ausführungsbeispiel der Erfindung ist insbesondere für Patienten mit Sick-Sinus Syndrom oder für Patienten mit chronischem Vorhofflimmern geeignet. Der Herzschrittmacher ist jedoch nicht für Patienten, die eine hohe Grundfrequenz benötigen (z.B. nach AV-Knotenablation oder Torsaden), für Patienten mit häufigen SVES und VES (Gefahr der Verlängerung der komp. Pause) oder für Patienten mit Carotis-Sinus-Syndrom (nicht ausreichende Anhebung der Frequenz während Episode) geeignet.

Die (Nacht-) Absenkung der Grundfrequenz bei Patientenruhe wird nicht an starre Zeiten geknüpft, sondern an die Aktivität des Patienten. Außerdem wird die Grundfrequenz nicht abrupt sondern schrittweise herabgesenkt.

Wird über einen langen Zeitraum keine Aktivität gemessen, so wird die Grundfrequenz schrittweise herabgesenkt. Wird innerhalb eines kurzen Zeitraumes Aktivität gemessen, so wird die Grundfrequenz wieder schrittweise auf ihren alten Wert angehoben.

Folgende Eingangswerte müssen von einem Arzt vorgegeben werden:

$GF_{UG}$        Minimal erlaubter Wert für die Grundfrequenz (untere Grenze)
$GF_{OG}$        Maximal erlaubter Wert für die Grundfrequenz (obere Grenze)
timer        Wiederholungswert des Algorithmus (Zeitdauer in Sekunden)
step        Änderungswert (1, 2 oder 5).

Damit ergibt sich beispielsweise für timer = 60 s:

Tabelle 1

| step | Zeitdauer für Absenkung um insgesamt 10ppm | Zeitdauer für Anhebung um insgesamt 10ppm |
|------|--------------------------------------------|-------------------------------------------|
| 1 | 20 min (10min + 10 min) | 11 min (1 min + 10 min) |
| 2 | 15 min (10min + 5 min) | 6 min (1 min + 5 min) |
| 5 | 12 min (10min + 2 min) | 3 min (1 min + 2 min) |

Parameter:

GF    Grundfrequenz

Hilfswerte (interne Variablen):

$GF_{level}$            "Level" der Grundfrequenz (aktueller Zustand)
Akt(t)            Aktueller Aktivitätsgrad (Wert von Aktivitäts-Erfassungseinheit 21) zum Zeitpunkt t
Mean_Akf'(t)    Mittelwert des Aktivitätsgrades der letzten n Minuten zum Zeitpunkt t
Min_Akt        Minimaler Aktivitätsgrad (min. Wert von Aktivitäts-Erfassungseinheit 21)
Max_Akt        Maximaler Aktivitätsgrad (max. Wert von Aktivitäts-Erfassungseinheit 21)
$Sum\_Akt^n$        Summe der Aktivitätswerte der letzten n Minuten

Initialisierungen:

$GF_{level} = 10$

Gemäß dem Wert von timer wird die folgende Auswertung von der Stimulations-Steuereinheit 22 durchgeführt:

Schritt 31:    $Sum\_Akt^{10} = Sum\_Akt^{10} + Mean\_Akt^1 (0) - Mean\_Akt^1(-10min)$

Schritt 32:    $Sum\_Akt^3 = Sum\_Akt^3 + Mean\_Akt^1 (0) - Mean\_Akt^1 (-3min)$

Schritt 33:    $if (Sum\_Akt^{10} \leq 11*Min\_Akt)$  and    falls (so gut wie) keine Aktivität in den letzten

$(GF_{level} > 0)$    10 Min. u. GF noch nicht vollständig abgesenkt{

Schritt 35:    $if (GF \geq GF_{UG} + step)$    Falls GF noch oberhalb des unteren

Grenzwertes    {

Schritt 36:    $GF = GF - step$    GF weiter absenken (insgesamt um 10ppm)

Schritt 37:    $GF_{level} = GF_{level} - step$    Absenkungslevel sichern

}

Schritt 38:    zu Schritt 43

}

Schritt 34:    $if (Sum\_Akt^3 > 4*Min\_Akt)$ and    Falls in den letzten 3 Min. eine (geringe)

Aktivität

$(GF_{level} < 10)$    und GF noch nicht vollständig angehoben

{

Schritt 39:    $if (GF \leq GF_{OG} - step)$

{

Schritt 40:    $GF = GF + step$    GF weiter anheben (insges. um 10ppm)

Schritt 41:    $GF_{level} = GF_{level} + step$    Absenkungslevel sichern

}

Schritt 42:    zu Schritt 43

}

Schritt 43:    Ende der Abarbeitung (erneuter Aufruf von Schritt 31 nach timer sec.)

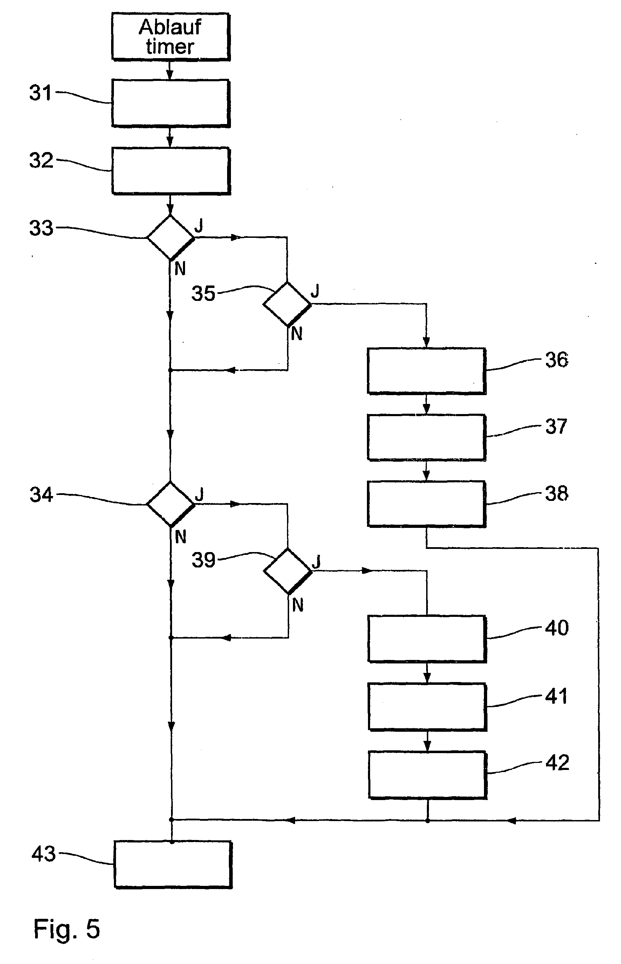

In Fig. 5 ist ein Flussdiagramm eines Algorithmus gemäß dem dritten Ausführungsbeispiel der Erfindung gezeigt. In Schritt 31 wird die Summe der Aktivitätswerte der letzten 10 Minuten gebildet, d. h. $Sum\_Akt^{10} = Sum\_Akt^{10} + Mean\_Akt^1 (0) - Mean\_Akt^1(-10min)$, und in Schritt 32 wird die Summe der Aktivitätswerte der letzten drei Minute gebildet, d. h. $Sum\_Akt^3 = Sum\_Akt^3 + Mean\_Akt^1 (0) - Mean\_Akt^1 (-3min)$. Falls in Schritt 33 (so gut wie) keine Aktivität in den letzten 10 Minuten erfasst und die Grundfrequenz $GF_{level}$ noch nicht vollständig abgesenkt wurde, schreitet der Fluss zu Schritt 35 weiter. Andernfalls schreitet der Fluss zu Schritt 34 weiter.

In Schritt 35 wird geprüft, ob die Grundfrequenz GF sich noch oberhalb des unteren Grenzwertes befindet. Wenn dies nicht der Fall ist, schreitet der Fluss zu Schritt 34 weiter. Andernfalls schreitet der Fluss zu Schritt 36 weiter. In Schritt 36 wird die Grundfrequenz GF um den Änderungswert step weiter abgesenkt (insgesamt um 10ppm) und der Fluss schreitet zu Schritt 37 weiter. In Schritt 37 wird das Absenkungs-Level gesichert und der Fluss schreitet zu Schritt 38 weiter. In Schritt 38 springt der Fluss zu Schritt 43.

In Schritt 34 wird geprüft, ob in den letzten 3 Minuten eine (geringe) Aktivität erfasst und die Grundfrequenz noch nicht vollständig angehoben wurde. Falls dies der Fall ist, schreitet der Fluss zu Schritt 39 weiter, andernfalls schreitet der Fluss zu Schritt 43 weiter.

In Schritt 39 wird geprüft, ob sich die Grundfrequenz GF noch unterhalb des oberen Grenzwertes $GF_{OG}$ befindet. Falls dies der Fall ist, schreitet der Fluss zu Schritt 40, andernfalls schreitet der Fluss zu Schritt 43 weiter. In Schritt 40 wird die Grundfrequenz GF um den Änderungswert step weiter angehoben (insgesamt um 10ppm) und der Fluss schreitet zu Schritt 41 weiter, wo das Absenkungs-Level gesichert wird. Anschließend schreitet der Fluss zu Schritt 42 weiter, bei dem der Fluss zu Schritt 43 geleitet wird.

In Schritt 43 endet der Ablauf. Nach Ablauf der festgesetzten Zeit (timer) wird der Ablauf erneut in Schritt 31 gestartet.

Dieser Algorithmus wird gem. dem Wert der Variablen timer z.B. alle 60 Sekunden wiederholt.

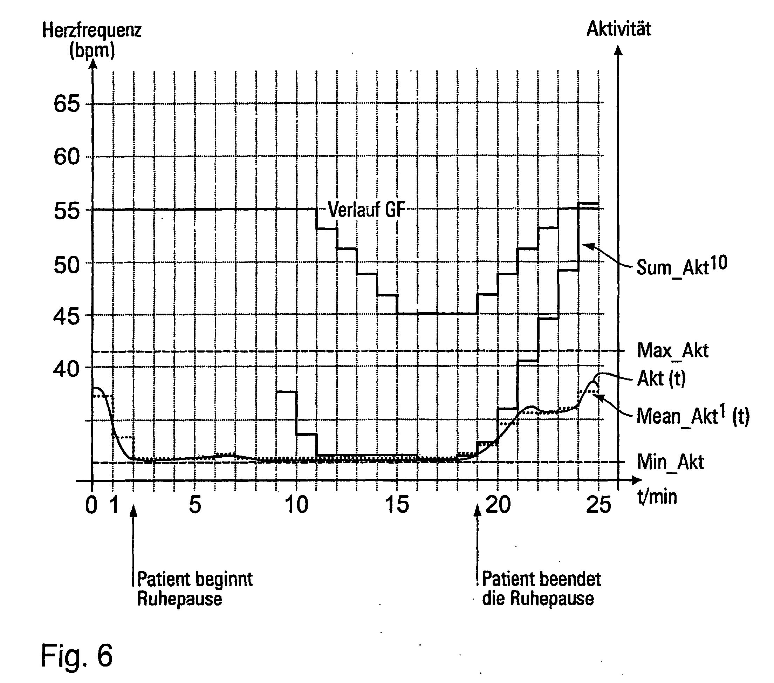

In Fig. 6 ist einen Graph der Herzfrequenz eines Patienten mit einem ratenadaptiven Herzschrittmacher gemäß dem dritten Ausführungsbeispiel von Figur 4, über die Zeit dargestellt. Eine Absenkung der Grundfrequenz während einer Ruhepause des Patienten sowie der Anstieg der Grundfrequenz am Ende der Ruhepause ist in diesem Graphen deutlich zu sehen.

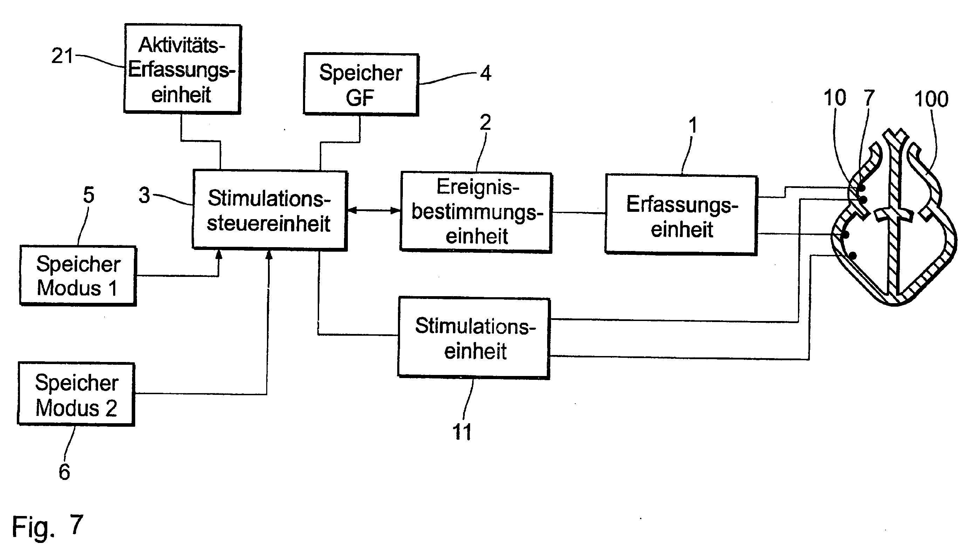

In Fig. 7 ist ein ratenadaptiver Herzschrittmacher gemäß einem vierten Ausführungsbeispiel der Erfindung gezeigt. Der Aufbau des Herzschrittmachers entspricht im Wesentlichen dem Aufbau des Herzschrittmachers gemäß dem ersten Ausführungsbeispiel der Erfindung aus Fig. 1. Zusätzlich zu dem in Fig. 1 gezeigten Herzschrittmacher weist der Herzschrittmacher gemäß dem vierten Ausführungsbeispiel der Erfindung eine Aktivitäts-Erfassungseinheit 21 auf, welche mit der Stimulations-Steuereinheit 3 verbunden ist.

Die Erfassungseinheit 1 erfasst die EKG-Signale des Herzens 100 anhand der Elektroden 7 und 8 und leitetet diese Signale an die Ereignis-Bestimmungseinheit 2 weiter. Die Ereignis-Bestimmungseinheit 2 empfängt die von der Erfassungseinheit 1 ausgegebenen EKG-Signale des Herzens sowie von der Stimulations-Steuereinheit 3 ausgegebene Signale, welche anzeigen, ob eine Stimulation stattgefunden hat. Anhand dieser empfangenen Signale bestimmt die Ereignis-Bestimmungseinheit 2, ob es sich bei einem erfassten Ereignis um ein spontanes Ereignis oder um ein stimuliertes Ereignis handelt. Das Ergebnis dieser Bestimmung wird an die Stimulations-Steuereinheit 3 weitergeleitet. Anhand dieses Ergebnisses wählt die Stimulations-Steuereinheit 3 den im Speicher 5 gespeicherten Modus 1 oder den im Speicher 6 gespeicherten Modus 2 aus.

Der ratenadaptiven Herzschrittmacher gemäß viertem Ausführungsbeispiel entspricht im wesentlichen einer Kombination der Merkmalen des Herzschrittmachers gemäß dem ersten Ausführungsbeispiel und einem Herzschrittmacher gemäß dem dritten Ausführungsbeispiel der Erfindung. Der wesentliche Unterschied besteht darin, dass der Pegel der Grundfrequenz in Abhängigkeit von der Aktivität des Patienten variiert wird.

Die Grundfrequenz GF wird möglichst dicht unter der Eigenfrequenz des Patienten gehalten. Dadurch greift der HSM standardmäßig nicht ein, es werden jedoch kompensatorische Pausen nach VES durch einen HSM-Stimulus unterbrochen. Gleichzeitig wird in Ruhepausen die minimale Grundfrequenz (unterer Grenzwert) schrittweise um insgesamt 10 ppm abgesenkt. Sobald eine Aktivität erkannt wird, wird die minimale Grundfrequenz rasch wieder angehoben.

Geeignete Patienten sind bevorzugt Patienten mit einem Herzschrittmacher im DDD oder AAI-Modus und mit häufigen ventrikulären und supraventrikulären Extrasystolen (z.B. bei Sick-Sinus-Syndrom, AV-Blockierungen oder HOCM)

Dies lässt sich beispielsweise bei Patienten mit einem AV-Block II°, Typ 2:1, dominierend, mit häufigen VES einsetzen.

Nicht geeignete Patienten sind Patienten mit höher eingestellter Grundfrequenz, z.B. nach AV-Knotenablation, Patienten mit hochgradiger Herzinsuffizienz ohne Schenkelblock, Patienten, bei denen ein hoher Stimulationsanteil gewünscht wird, z.B. bei Torsaden, Patienten mit einem HSM im VDD- und VVI-Modus, da es hier bei erhaltenem Vorhofrhythmus zur asynchronen Ventrikelstimulation kommt, und Patienten mit Carotis-Sinus-Syndrom (nicht ausreichende Anhebung der Frequenz während Episode)

Ein Nachführen der Grundfrequenz an die aktuelle intrinsische Herzfrequenz soll die kompensatorischen Pausen verringern, ohne in größeren Maße den intrinsischen Herzfrequenzverlauf zu stören. Dies ist besonders bei den Patienten interessant, bei denen eine niedrige Grundfrequenz programmiert wurde und hierdurch lange kompensatorische Pausen entstehen können, ohne dass der Schrittmacher einschreiten kann.

Gleichzeitig wird die (Nacht-) Absenkung der Grundfrequenz bei Patientenruhe nicht an starre Zeiten geknüpft, sondern an die Aktivität des Patienten. Außerdem wird die Grundfrequenz nicht abrupt sondern schrittweise herabgesenkt.

Die Grundfrequenz wir so nachgeführt, dass sie möglichst knapp unterhalb der intrinsischen Herzfrequenz liegt. Insgesamt sollte - ausgenommen in den kompensatorischen Pausen nach Extrasystolen - möglichst wenig stimuliert werden.

Gleichzeitig wird dafür gesorgt, dass die Grundfrequenz stets innerhalb der definierten Grenzen, d. h. Grundfrequenz OG als minimal erlaubter Wert für die Grundfrequenz $GF_{OG}$ und Grundfrequenz UG + 10 ppm (bei Aktivität) bzw. Grundfrequenz UG (bei Ruhe) liegt.

Wird über einen langen Zeitraum keine Aktivität gemessen, so wird die untere Grenze schrittweise um insgesamt 10ppm herabgesenkt, so dass die Grundfrequenz ebenfalls weiter abgesenkt werden kann. Wird innerhalb eines kurzen Zeitraumes Aktivität gemessen, so wird die untere Grenze wieder schrittweise auf ihren alten Wert angehoben, wodurch auch die nachgeführte Grundfrequenz diese niedrigen Werte nicht mehr annehmen kann.

Folgende Eingangswerte müssen von einem Arzt patientenspezifisch vorgegeben werden:

$GF_{OG}$      *Minimal erlaubter Wert für die Grundfrequenz (obere Grenze)*
$GF_{UG}$      *Minimal erlaubter Wert für die Grundfrequenz (untere Grenze)*
*delta_ap      Änderungswert nach Ap-Event*
*delta_as      Änderungswert nach As-Event*
*delta_ves      Änderungswert nach VES-Event*
*timer          Wiederholung des Algorithmus (Zeitdauer in Sekunden)*
*step          Änderungswert (1, 2 oder 5).*

Damit ergibt sich beispielsweise für timer = 60 s:

*Tabelle 2*

| Step | Zeitdauer für Absenkung um insgesamt 10ppm | Zeitdauer für Anhebung um insgesamt 10ppm |
|:---:|:---:|:---:|
| 1 | 20 min (10min + 10 min) | 11 min (1 min + 10 min) |
| 2 | 15 min (10min + 5 min) | 6 min (1 min + 5 min) |
| 5 | 12 min (10min + 2 min) | 3 min (1 min + 2 min) |

Parameter:

*GF      Grundfrequenz*

Hilfswerte (interne Variablen):

$GI$                *Grundintervall*
$GI_{UG}$              *Minimal erlaubter Wert für das Grundintervall (untere Grenze)*
$GI_{OG}$              *Maximal erlaubter Wert für das Grundintervall (obere Grenze)*
*ASI                letztes atriales Spontanintervall (intrinsisch und/oder stimuliert)*
*V_VES_I            Intervall zw. letztem ventr. Ereignis (intr. Oder stim.) und detektierter VES*
*AV-Delay            HSM-Parameter*
$GF_{UG,var}$          *Minimal erlaubter Wert für die Grundfrequenz (untere Grenze bei Aktivität)*
$GF_{level}$          *"Level" der Grundfrequenz (aktueller Zustand)*
*Akt(t)              Aktueller Aktivitätsgrad (Wert von Aktivitäts-Erfassungseinheit 21) zum Zeitpunkt t*
$Mean\_Akt^n(t)$    *Mittelwert des Aktivitätsgrades der letzten n Minuten zum Zeitpunkt t*
*Min_Akt            Minimaler Aktivitätsgrad (min. Wert von Aktivitäts-Erfassungseinheit 21)*
*Max_Akt            Maximaler Aktivitätsgrad (max. Wert von Aktivitäts-Erfassungseinheit 21)*
$Sum\_Akt^n$        *Summe der Aktivitätswerte der letzten n Minuten*

Initialisierungen:

$$GF_{level} = 10$$

$$GF_{UG,var} = GF_{UG} + 10\ ppm$$

$$GI_{OG} = 60000 / GF_{UG,var}$$

$$GI_{UG} = 60000 / GF_{OG}$$

Nach jeder Vorhofaktion wird die folgende Auswertung gestartet:

$$Falls\ Ap \quad \Rightarrow \quad GI = GI + delta\_ap$$

$$Falls\ As \quad \Rightarrow \quad GI = ASI + delta\_as$$

$$Falls\ VES \quad \Rightarrow \quad GI = AV\text{-}Delay + V\_VES\_I + delta\_ves$$

$$Falls\ (GI < GI_{UG}) \qquad GI = GI_{UG}$$

$$Falls\ (GI > GI_{OG}) \qquad GI = GI_{OG}$$

Gemäß dem Wert von timer wird die folgende Auswertung durchgeführt:

*Schritt 51:* $Sum\_Akt^{10} = Sum\_Akt^{10} + Mean\_Akt^1$ (0) - $Mean\_Akt^1(-10min)$

*Schritt 52:* $Sum\_Akt^3 = Sum\_Akt^3 + Mean\_Akt^1$ (0) - $Mean\_Akt^1$ (-3min)

*Schritt 53:* *if ($Sum\_Akt^{10} \leq 11*Min\_Akt$) and*     Falls (so gut wie) keine Aktivität in den letzten

        *($GF_{level} > 0$)*         10 Min. u. GF noch nicht vollständig abge-

     senkt

       *{*

*Schritt 55:*      *if ($GF_{UG,var} \geq GF_{UG} + step$)*     Falls GF noch oberhalb des unteren Grenzwertes

        *{*

*Schritt 56:*        *$GF_{UG,var} = GF_{UG,var} - step$*    GF weiter absenken (insgesamt um 10ppm)

*Schritt 57:*        *$GF_{level} = GF_{level} - step$*     Absenkungslevel sichern

        *}*

       *zu Schritt 61*

      *}*

*Schritt 54:* *if ($Sum\_Akt^3 > 4*Min\_Akt$) and*      Falls in den letzten 3 Min. eine (geringe)

Aktivität

       *($GF_{level} < 10$)*        und GF noch nicht vollständig angehoben

       *{*

*Schritt 58:*      *if ($GF_{UG,var} \leq GF_{OG} - step$)*    Falls GF noch unterhalb des oberen Grenzwertes          *{*

*Schritt 59:*        *$GF_{UG,var} = GF_{UG,var} + step$*   GF weiter anheben (insgesamt um 10ppm)

*Schritt 60:*        *$GF_{level} = GF_{level} + step$*    Absenkungslevel sichern

        *}*

       *zu Schritt 61*

      *}*

*Schritt 61:* $Gl_{OG} = 60000 / GF_{UG,var}$       Änderungen für Beat-to-Beat Algorihmus

    über-

         nehmen

*Schritt 62:* *Ende der Abarbeitung (erneuter Aufruf von Schritt 51 nach timer sec.)*

In Fig. 7 ist ein Flussdiagramm eines Algorithmus gemäß dem vierten Ausführungsbeispiel der Erfindung gezeigt. In Schritt 51 wird die Summe der Aktivitätswerte der letzten 10 Minuten gebildet, d. h. $Sum\_Akt^{10} = Sum\_Akt^{10} + Mean\_Akt^1$ (0) - $Mean\_Akt^1$(-10min), und in Schritt 52 wird die Summe der Aktivitätswerte der letzten drei Minuten gebildet, d. h. $Sum\_Akt^3 = Sum\_Akt^3 + Mean\_Akt^1$ (0) - $Mean\_Akt^1$ (-3min). Falls in Schritt 53 (so gut wie) keine Aktivität in den letzten 10 Minuten erfasst und die Grundfrequenz $GF_{level}$ noch nicht vollständig abgesenkt wurde, schreitet der Fluss zu Schritt 55 weiter. Andernfalls schreitet der Fluss zu Schritt 54 weiter.

In Schritt 55 wird geprüft, ob die Grundfrequenz GF sich noch oberhalb des unteren Grenzwertes befindet. Wenn dies nicht der Fall ist, schreitet der Fluss zu Schritt 54 weiter. Andernfalls schreitet der Fluss zu Schritt 56 weiter. In Schritt 56 wird die Grundfrequenz GF um den Änderungswert step weiter abgesenkt (insgesamt um 10ppm) und der Fluss schreitet zu Schritt 57 weiter. In Schritt 57 wird das Absenkungs-Level gesichert und der Fluss schreitet zu Schritt 61 weiter.

In Schritt 54 wird geprüft, ob in den letzten 3 Minuten eine (geringe) Aktivität erfasst und die Grundfrequenz GF noch nicht vollständig angehoben wurde. Falls dies der Fall ist, schreitet der Fluss zu Schritt 58 weiter, andernfalls schreitet der Fluss zu Schritt 61 weiter.

In Schritt 58 wird geprüft, ob sich die Grundfrequenz GF noch unterhalb des oberen Grenzwertes $GF_{OG}$ befindet. Falls dies der Fall ist schreitet der Fluss zu Schritt 59, andernfalls schreitet der Fluss zu Schritt 61 weiter. In Schritt 59 wird die Grundfrequenz GF um den Änderungswert step weiter anhoben (insgesamt um 10ppm) und der Fluss schreitet zu Schritt 60 weiter, wo das Absenkungs-Level gesichert wird. Anschließend schreitet der Fluss zu Schritt 61 weiter. In Schritt 61 wird die Änderung der Obergrenze des Grundintervalls $GI_{OG}$ für den Beat-to-Beat Algorithmus übernommen.

In Schritt 62 endet der Ablauf. Nach Ablauf einer vorgegebenen Zeit (timer) wird der Ablauf erneut in Schritt 51 gestartet.

Dieser Algorithmus wird gemäß dem Wert der Variablen timer z.B. alle 60 Sekunden wiederholt.

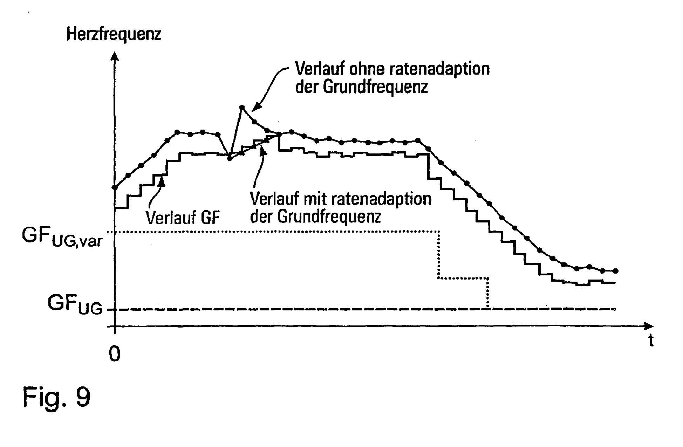

In Fig. 9 ist der Frequenzverlauf eines Herzens über die Zeit für einen Herzschrittmacher gemäß dem vierten Ausführungsbeispiel gezeigt. Der Verlauf der Herzfrequenz ohne Ratenadaption der Grundfrequenz ist durch Punkte und der Verlauf der ratenadaptierten Grundfrequenz GF ist als eine durchgezogene Linie dargestellt. In Fig. 9 sind ferner die obere und die untere Grundfrequenz $GF_{UG}$, $GF_{OG}$ sowie die variable untere Grundfrequenz $GF_{UG,var}$ gezeigt. Beachtenswert ist dabei insbesondere, wie die variable untere Grundfrequenz $GF_{UG,var}$ mit fortschreitender Zeit und ausbleibender Aktivität abgesenkt wird.

## Patentansprüche

1. Ratenadaptiver Herzschrittmacher, mit

   - einer Erfassungseinheit (1),
   - einer Ereignis-Bestimmungseinheit (2),
   - einer Stimulations-Steuereinheit (3) und
   - einer Stimulationseinheit (11),

   wobei die Ereignis-Bestimmungseinheit (2) mit der Erfassungseinheit (1) und Stimulations-Steuereinheit (3) verbunden sowie die Stimulationseinheit (11) mit der Stimulations-Steuereinheit (3) verbunden ist, wobei die Erfassungseinheit (1) dazu ausgebildet ist, EKG-Signale eines Herzens (100) zu erfassen, wobei die Ereignis-Bestimmungseinheit (2) dazu ausgebildet ist, anhand der von der Erfassungseinheit (1) erfassten EKG-Signale und von der Stimulations-Steuereinheit (3) ausgesendeten Signalen zu bestimmen, ob ein erfasstes Ereignis ein spontanes oder ein stimuliertes Ereignis darstellt, und wobei die Stimulations-Steuereinheit (3) dazu ausgestaltet ist, eine Stimulationsrate in Abhängigkeit von einer Grundrate und einem physiologischen Bedarf zu bestimmen und die Grundrate bei jedem erfassten natürlichen oder jedem oder jedem n-ten stimulierten Ereignis in Abhängigkeit von der Ereignis-Bestimmungseinheit (2) in einem ersten Modus bei Auftreten von atrialen Ereignissen derart zu verändern, dass in jedem Falle eines spontanen atrialen Ereignisses ein Spontanintervall um einen ersten Änderungswert verlängert wird und dass in jedem Falle eines stimulierten atrialen Ereignisses ein Grundintervall um einen zweiten Änderungswert verlängert wird, wodurch in beiden Fällen die Grundrate gesenkt wird, sowie alternativ oder zusätzlich in einem zweiten Modus bei Auftreten von ventrikulären Ereignissen die Grundrate derart zu verändern, dass in jedem Falle eines spontanen ventrikulären Ereignisses die Grundrate um einen dritten Änderungswert erhöht wird und in jedem n-ten aufeinanderfolgenden Falle eines stimulierten ventrikulären Ereignisses die Grundrate um einen vierten Änderungswert, der kleiner ist als der dritte Änderungswert, gesenkt wird, wobei die Stimulationseinheit (11) dazu ausgestaltet ist, das Herz (100) entsprechend der von der Stimulations-Steuereinheit (3) empfangenen Signalen zu stimulierten, wobei n eine ganze Zahl größer oder gleich 1 ist.

2. Ratenadaptiver Herzschrittmacher nach Anspruch 1, bei dem in dem ersten Modus bei Auftreten von ventrikulären Ereignissen die Grundrate derart verändert wird, dass bei Auftreten einer ventrikulären Extrasystole (VES) ein Grundintervall um einen fünften Änderungswert (delta_ves) verlängert wird, wodurch die Grundrate im Falle ventrikulärer Extrasystolen gesenkt wird.

3. Ratenadaptiver Herzschrittmacher nach Anspruch 1 oder 2, wobei die Stimulations-Steuereinheit (3) dazu ausgestaltet ist, die Grundrate des Herzens (100) innerhalb vorgegebener Grenzen zu bestimmen.

4. Ratenadaptiver Herzschrittmacher nach Anspruch 1, wobei die Stimulations-Steuereinheit (3) dazu ausgestaltet ist, die Stimulationseinheit (11) in Abhängigkeit von jedem atrialen oder ventrikulären Ereignis zu steuern.

5. Ratenadaptiver Herzschrittmacher nach Anspruch 1, mit einer Aktivitäts-Erfassungseinheit (21), welche dazu ausgestaltet ist, die Aktivität eines Patienten zu erfassen und ein entsprechendes Aktivitätssignal auszugeben, wobei die Stimulations-Steuereinheit (4) mit der Aktivitäts-Erfassungseinheit (21) verbunden und dazu ausgebildet ist, eine Grundrate eines Patienten entsprechend einer ersten oder einer zweiten Betriebsart derart ratenadaptiv zu bestimmen, dass die Grundrate des Herzens (100) entsprechend der ersten Betriebsart in einem vorgegebenen ersten Schrittintervall (N1) abgesenkt wird, wenn innerhalb eines vorgegebenen ersten Zeitintervalls (T1) das Aktivitätssignal keine Aktivität des Patienten anzeigt, und die Grundrate des Herzens (100) entsprechend der zweiten Betriebsart in einem vorgegebenen zweiten Schrittintervall (N2) erhöht wird, wenn innerhalb eines vorgegebenen zweiten Zeitintervalls (T2) das Aktivitätssignal eine Aktivität des Patienten anzeigt, wobei das erste Zeitintervall (T1) länger als das zweite Zeitintervall (T2) ist, wobei das erste Schrittintervall (N1) gleich dem zweiten Schrittintervall (N2) ist.

**Claims**

1.  A rate-adaptive cardiac pacemaker, comprising

    • an acquisition unit (1);
    • an event determination unit (2);
    • a stimulation control unit (3);
    • and a stimulation unit (11);

    wherein the event determination unit (2) is connected to the acquisition unit (1) and to the stimulation control unit (3), and the stimulation unit (11) is connected to the stimulation control unit (3), wherein the acquisition unit (1) is designed to acquire ECG signals of a heart (100), wherein the event determination unit (2) on the basis of the ECG signals acquired by the acquisition unit (1) and on the basis of signals emanating from the stimulation control unit (3) is designed to determine whether an acquired event represents a spontaneous or a stimulated event, and wherein the stimulation control unit (3) is designed to determine a stimulation rate depending on a base rate and a physiological requirement, and to change the base rate during every acquired natural event or during every or every nth stimulated event depending on the event determination unit (2) in a first mode if atrial events occur, such that in each case of a spontaneous atrial event a spontaneous interval is extended by a first change value; and that in each case of a stimulated atrial event a base interval is extended by a second change value, as a result of which in both cases the base rate is decreased, and, as an alternative or in addition, in a second mode in the case of a ventricular event occurring the base rate is changed in such a way that in each case of a spontaneous ventricular event the base rate is increased by a third change value and in each nth subsequent case of a stimulated ventricular event the base rate is decreased by a fourth change value which is smaller than the third change value, wherein the stimulation unit (11) is designed to stimulate the heart (100) according to the signals received from the stimulation control unit (3), wherein "n" denotes a integer larger than 1, or 1.

2.  The rate-adaptive cardiac pacemaker according to claim 1, in which in the first mode, if a ventricular event occurs, the base rate is changed in such a way that if a ventricular premature atrial contraction (VES) occurs, a base interval is extended by a fifth change value (delta_ves), as a result of which the base rate is decreased in the case of ventricular premature atrial contractions.

3.  The rate-adaptive cardiac pacemaker according to claim 1 or 2, wherein the stimulation control unit (3) is designed to determine the base rate of the heart (100) within specified limits.

4.  The rate-adaptive cardiac pacemaker according to claim 1, wherein the stimulation control unit (3) is designed to control the stimulation unit (11) depending on each atrial or ventricular event.

5.  The rate-adaptive cardiac pacemaker according to claim 1, comprising an activity acquisition unit (21) that is designed to acquire the activity of a patient and to emit a corresponding activity signal, wherein the stimulation control unit (4) is connected to the activity acquisition unit (21) and is designed to determine a base rate of a patient according to a first or a second operating mode in a rate-adaptive manner in such a way that the base rate of the heart (100) is reduced according to the first operating mode in a predefined first step interval (N1) if within a predefined first time interval (T1) the activity signal does not show any patient activity, and the base rate of the heart (100) according to the second operating mode in a predefined second step interval (N2) is increased if within a predefined second time interval (T2) the activity signal shows patient activity, wherein the first time interval (T1) is longer than the second time interval (T2), wherein the first step interval (N1) is of the same length as the second step interval (N2).

**Revendications**

1.  Pacemaker cardiaque à fréquence adaptable, comportant

- une unité d'enregistrement (1),
- une unité de détermination de phénomène (2),
- une unité de contrôle de stimulation (3) et
- une unité de stimulation (11),

dans lequel l'unité de détermination de phénomène (2) est reliée à l'unité d'enregistrement (1) et à l'unité de stimulation (11) et l'unité de stimulation (11) à l'unité de contrôle de stimulation (3), l'unité d'enregistrement (1) étant conçue pour enregistrer des signaux ECG d'un coeur (100), l'unité de détermination de phénomène (2) étant conçue pour, sur la base des signaux ECG enregistrés par l'unité d'enregistrement (1) et des signaux émis par l'unité de contrôle de stimulation (3), déterminer si un phénomène enregistré constitue un phénomène spontané ou un phénomène stimulé, et l'unité de contrôle de stimulation (3) étant conçue pour déterminer une fréquence de stimulation en fonction d'une fréquence de base et d'un besoin physiologique et, à chaque phénomène naturel ou à chaque ou chaque énième phénomène stimulé enregistré, en fonction de l'unité de détermination de phénomène (2), dans un premier mode, en cas de survenance de phénomènes atriaux, pour modifier la fréquence de base de manière à ce que, dans chaque cas d'un phénomène atrial spontané, un intervalle spontané soit prolongé d'une première valeur de modification et que, dans chaque cas d'un phénomène atrial stimulé, un intervalle de base soit prolongé d'une deuxième valeur de modification, ce qui fait dans les deux cas baisser la fréquence de base, ainsi qu'en alternative ou en complément, dans un deuxième mode, en cas de survenance de phénomènes ventriculaires, à modifier la fréquence de base de manière à ce que, dans chaque cas d'un phénomène ventriculaire spontané, la fréquence de base soit augmentée d'une troisième valeur de modification et, dans chaque énième cas successif d'un phénomène ventriculaire stimulé, la fréquence de base soit abaissée d'une quatrième valeur de modification qui est inférieure à la troisième valeur de modification, tandis que l'unité de stimulation (11) est conçue pour stimuler le coeur (100) en fonction des signaux reçus par l'unité de contrôle de stimulation (3), n étant un nombre entier supérieur ou égal à 1.

2. Pacemaker cardiaque à fréquence adaptable selon la revendication 1, dans lequel, dans le premier mode, en cas de survenance de phénomènes ventriculaires, la fréquence de base est modifiée de manière à ce qu'en cas de survenance d'une extrasystole ventriculaire (VES), un intervalle de base soit prolongé d'une cinquième valeur de modification (delta_ves), ce qui fait diminuer la fréquence de base dans le cas d'extrasystoles ventriculaires.

3. Pacemaker cardiaque à fréquence adaptable selon la revendication 1 ou 2, dans lequel l'unité de contrôle de stimulation (3) est conçue pour déterminer la fréquence de base du coeur (100) dans des limites prédéfinies.

4. Pacemaker cardiaque à fréquence adaptable selon la revendication 1, dans lequel l'unité de contrôle de stimulation (3) est conçue pour contrôler l'unité de stimulation (11) en fonction de chaque phénomène atrial ou ventriculaire.

5. Pacemaker cardiaque à fréquence adaptable selon la revendication 1, comprenant une unité d'enregistrement d'activité (21) qui est conçue pour enregistrer l'activité d'un patient et émettre un signal d'activité correspondant, dans lequel l'unité de contrôle de stimulation (4) est reliée à l'unité d'enregistrement d'activité (21) et est conçue pour déterminer une fréquence de base d'un patient en fonction d'un premier ou d'un deuxième mode de fonctionnement de manière à ce que la fréquence de base du coeur (100) soit diminuée en fonction du premier mode de fonctionnement pendant un premier intervalle prédéfini entre les étapes (N1) si, pendant un premier intervalle de temps prédéfini (T1), le signal d'activité n'indique pas d'activité du patient et que la fréquence de base du coeur (100) soit augmentée en fonction du deuxième mode de fonctionnement pendant un deuxième intervalle prédéfini entre les étapes (N2) si, pendant un deuxième intervalle de temps prédéfini (T2), le signal d'activité indique une activité du patient, le premier intervalle de temps (T1) étant plus long que le deuxième intervalle de temps (T2), tandis que le premier intervalle entre les étapes (N1) est égal au deuxième intervalle entre les étapes (N2).

Fig. 1

EP 1 291 037 B1

Fig. 2

Fig. 3

EP 1 291 037 B1

| 21 | | 22 | | 24 | 100 |
|---|---|---|---|---|---|
| Aktivitäts-Erfassungs-einheit | | Stimulations-steuereinheit | | | |

Stimulations-einheit

23

25

Fig. 4

Fig. 5

Fig. 6

EP 1 291 037 B1

**Fig. 7**

| | |
|---|---|
| 21 — Aktivitäts-Erfassungs-einheit | Speicher GF — 4 |
| | 2 |
| | Ereignis-bestimmungs-einheit |
| 5 — 3 Stimulations-steuereinheit | 1 Erfassungs-einheit |
| Speicher Modus 1 | Stimulations-einheit |
| Speicher Modus 2 | 11 |
| 6 | |

10  7  100

EP 1 291 037 B1

Fig. 8

Fig. 9